# EUROPÄISCHE PATENTSCHRIFT

(11) **EP 2 094 323 B1**
(45) Veröffentlichungstag und Bekanntmachung des Hinweises auf die Patenterteilung: **23.11.2011**
(21) Anmeldenummer: 07817688.0
(22) Anmeldetag: 16.10.2007
(51) Int. Cl.: A61L 27/46, A61L 24/10

(54) **BEHANDLUNG VON OSTEOPOROSE**
TREATMENT OF OSTEOPOROSIS
TRAITEMENT DE L'OSTÉOPOROSE

(30) Priorität: 16.10.2006 DE 102006048833; 02.02.2007 WO PCT/DE2007/000192
(43) Veröffentlichungstag der Anmeldung: 02.09.2009
(73) Patentinhaber: Universität Rostock, 18055 Rostock (DE)
(72) Erfinder: GRADL, Georg, 18055 Rostock (DE); BEHREND, Detlef, 18119 Warnemünde (DE); SCHMITZ, Klaus-Peter, 18119 Warnemünde (DE); STERNBERG, Katrin, 18055 Rostock (DE); SCHMOHL, Kathleen, 18055 Rostock (DE); KRAMER, Sven, 18069 Rostock (DE)
(74) Vertreter: UEXKÜLL & STOLBERG
(86) Internationale Anmeldenummer: PCT/DE2007/001853
(87) Internationale Veröffentlichungsnummer: WO 2008/046405

(56) Entgegenhaltungen:
- EP-A- 1 518 569
- WO-A-2007/090373
- US-A- 5 583 114
- US-A1- 2004 249 015

## Beschreibung

Die vorliegende Erfindung betrifft eine Kombination von Komponenten zur Behandlung von Osteoporose und/oder zur Prophylaxe und Behandlung von Knochenbrüchen, welche Kollagen, zusätzliches Oligopeptid oder Protein, das mindestens zwei reaktive Aminogruppen und/oder Hydroxylgruppen umfasst, eine Calcium-haltige Substanz und ein Vernetzungsmittel umfasst, wobei das Vernetzungsmittel ein reaktionfähiges endständig funktionalisiertes Oligolacton umfasst. Gegenstand der Erfindung sind auch aus der Kombination hergestellte künstliche Knochen und Implantate und die Verwendung der Kombination zur Fixierung von Implantaten sowie zur Behandlung von Osteoporose und/oder Prophylaxe und Behandlung von Knochenbrüchen. Ferner betrifft die Erfindung ein Verfahren zur Herstellung künstlicher Knochen und Implantate.

Osteoporose ist eine Erkrankung, die zu einem übermäßigen Abbau der Knochensubstanz und -struktur führt und mit erhöhter Frakturanfälligkeit einhergeht, die das ganze Skelett betrifft. Zunächst verläuft Osteoporose unmerklich, im Fall von Knochenbrüchen bedeutet die Erkrankung jedoch, insbesondere bei alten Menschen, eine hohe Krankheitsbelastung mit Schmerzen, Bettlägerigkeit und manchmal dauerhafter Immobilisierung. Osteoporose ist ein häufiges Krankheitsbild. Allein in Deutschland leiden ca. 8 Millionen Menschen darunter.

Man unterscheidet zwischen primärer und sekundärer Osteoporose. Zur primären Osteoporose zählen die postmenopausale Osteoporose und die Altersosteoporose (Involutionsosteoporose). Fast die Hälfte aller Frauen im Alter über 50 Jahren zeigen eine Reduktion der Knochendichte, wovon wiederum 50% eine manifeste Osteoporose entwickeln. Für Männer ab dem 70. Lebensjahr ist die Altersosteoporose ein ebenso häufiges Krankheitsbild.

Sekundäre Osteoporose tritt unter anderem als Folge von Stoffwechselerkrankungen oder hormonellen Störungen auf.

Osteoporotischer Knochen bricht bei geringer Belastung, wie Minimaltraumen oder schon durch die Last des Körpergewichts des Menschen. Die Fraktur ist schwer zu therapieren. Knochenbrüche finden sich bei Osteoporose vor allem am Oberschenkelhals, am Handgelenk und an den Wirbelkörpern der Wirbelsäule. Kompressions-Frakturen der Wirbelkörper (vertebral compression fractures, VCFs) sind z.B. häufig. Sie gehen mit einer Abnahme der Wirbelkörpergröße von mindestens 15% einher. Bei 84% solcher Frakturen treten Schmerzen auf, insbesondere intensive Schmerzen am Ort der Fraktur, die ungefähr 4 bis 6 Wochen anhalten. Oft kommt es zu chronischen Schmerzen, wenn eine Schicht stark zusammengefallen ist oder mehrere Schichten komprimiert sind. Lungenprobleme, Verlust der Beweglichkeit und geringere Belastbarkeit sind allgemeine Folgen. Damit verbunden sind oft chronische Depressionen, welche die mit der Verformung einhergehenden chronischen Schmerzen verschlimmern können. Die Folgen von Frakturen können vor allem bei Älteren nachhaltig sein. Durch Folgeerkrankungen wie Lungenentzündung oder Lungenembolie führen sie oft zum Tod.

Medikamente wie Calcium und Vitamin D sowie Biphosphonate sind in der Lage, die Knochensubstanz langsam zu stärken. Bei schwerer Osteoporose dauert dies lange und kann nicht lokal auf besonders frakturgefährdete Knochenbezirke wirken. Auch durch Bewegung kann der Osteoporose entgegengewirkt werden, da durch die bei Bewegung auftretenden Maximalkräfte die Knochenmasse und vor allem die Knochenfestigkeit vergrößert wird. Auch dies ist ein langsamer Prozess, und bei schwerer Osteoporose muss die zum Teil eingeschränkte Beweglichkeit und das Frakturrisiko beachtet werden.

Frakturen bei Osteoporose, insbesondere Wirbelkörperfrakturen, können mit minimalinvasiven Techniken behandelt werden, wie Vertebroplastie und Kyphoplastie. Hier ist es üblich, den Knochen mit dünnen Nadeln zu punktieren und mit einem zunächst breiig-flüssigen Material zu befüllen, welches im Inneren des Knochens aushärtet und diesen dann von innen stabilisiert (Vertebroplastie). Hierzu wird Knochenzement in verschiedenen Ausführungen bereitgehalten (z.B. Palacos), seit kurzem auch biodegradierbarer Zement. Im allgemeinen wird hierzu das synthetische Polymer Polymethylmethacrylat (PMMA) verwendet. Kyphoplastie ist die Verwendung von Ballonen, die in einem zusammengefallenen Wirbelkörper entfaltet werden können, so dass vor der Injektion von Zement eine Höhlung hergestellt wird. Die Stabilisierung von Frakturen mit diesen Verfahren führt oft zu einer Abnahme der Schmerzen. Die Risiken sind gering, aber es treten dennoch ernste Komplikationen auf. So kann PMMA mit systemischen Konsequenzen aus den Wirbelkörpern wieder austreten. Es kann zu Kompressionen des Rückenmarks oder der Nerven, Embolien in Venen und Lunge, bis hin zum vollständigen Kreislaufzusammenbruch kommen (Burton et al., 2003, Vertebroplasty and Kyphoplasty, Pain Physician 6:335-343).

Daher besteht ein Bedarf, alternative Verfahren und Präparate zur Stabilisierung von Knochen bei Osteoporose zu entwickeln, insbesondere solche, die gut verträglich sind und auch zur Prophylaxe von Knochenbrüchen eingesetzt werden können.

Dieses Problem wird durch die vorliegende Erfindung, gemäss den Gegenstand der Ansprüche, gelöst.

Insbesondere stellt die vorliegende Erfindung eine Kombination zur Behandlung von Osteoporose und/oder zur Prophylaxe und Behandlung von Knochenbrüchen zur Verfügung, welche mindestens ein Oligopeptid oder Protein, das mindestens zwei reaktive Aminogruppen and/oder Hydroxylgruppen umfasst, insbesondere Kollagen, eine Calcium-haltige Substanz und ein Vernetzungsmittel umfasst. Das Vernetzungsmittel umfasst ein reaktionsfähiges endständig funktionalisiertes Oligolacton als Brückenmolekül. Das Vernetzungsmittel umfasst in einer Ausführungsform ferner einen Katalysator der Vernetzungsreaktion. Das Vernetzungsmittel ist dazu geeignet, das Kollagen und gegebenenfalls weitere Substrate, die geeignete funktionelle Gruppen, insbesondere Aminogruppen umfassen (z.B. Oligopeptide) zu vernetzen.

Kollagen ist als Strukturprotein des menschlichen und tierischen Knochens in Form einer Tripelhelix wohlbekannt. Das im Rahmen der Erfindung verwendete Kollagen kann ein Kollagenhydrolysat sein, z.B. Gelatine. Es kann rekombinant hergestellt oder tierischen Ursprungs sein, etwa Rinder- oder Schweinekollagen. Bevorzugt wird ein Kollagenpulver eingesetzt, das Kollagen kann jedoch auch bereits als Suspension oder Lösung oder als vorgeformte Matrix vorliegen. Bevorzugt umfasst das Präparat 10-70 Gew-% Kollagen, insbesondere 20-50 Gew-% Kollagen oder 30 Gew-% Kollagen (die Prozentangaben sind bezogen auf die Gesamtmasse ohne Lösungsmittel).

Kollagen enthält eine Vielzahl von funktionellen Gruppen, z.B. Aminogruppen von Lysin, die vernetzt werden können.

Um die Vernetzung und damit Aushärtung des Präparats zu beschleunigen, wird ein weiteres Biopolymer zugesetzt, das eine Vielzahl funktioneller, eine Vernetzung ermöglichender Gruppen enthält, z.B. freier Aminogruppen. Vorteilhafterweise ist das Biopolymer nicht-toxisch und/oder biologisch abbaubar. Das Biopolymer ist ein Protein oder Oligopeptid, wobei diese Ausdrücke im Rahmen der vorliegenden Erfindung synonym verwendet werden. Das Protein oder Oligopeptid umfasst mindestens zwei Aminogruppen und/oder Hydroxylgruppen, insbesondere mindestens eine, vorzugsweise zwei oder mehr Diaminosäuren und/oder Hydroxylgruppen.

Im Rahmen der vorliegenden Erfindung wird als Peptid sowohl ein Oligopeptid (2 bis 100 Aminosäuren Länge) als auch ein Protein (Eiweiß, 100 bis 5000 Aminosäuren Länge, bevorzugt von 100-1000 oder 100-200 Aminosäuren Länge) bezeichnet. Bevorzugt weist das Peptid eine Länge von 4-100 Aminosäuren, insbesondere 10-20 Aminosäuren auf. Das Peptid kann ein Molekulargewicht von 1 kDa bis 100 kDa oder bis 200 kDa, insbesondere 2 kDa bis 50 kDa oder 5 kDa bis 20 kDa aufweisen. Es kann modifiziert bzw. substituiert sein, z.B. glykosyliert. Neben den üblichen proteinogenen Aminosäuren können auch modifizierte bzw. untypische Aminosäuren wie Hydoxylysin in dem Oligopeptid enthalten sein. Der Einsatz von D-Aminosäuren anstelle von L-Aminosäuren oder zusätzlich dazu ist möglich und verlangsamt den Abbau des Peptids.

Selbstverständlich können auch mehrere verschiedene derartige Peptide oder weitere Biopolymere enthalten sein. Diese Variante ist in der Folge nicht gesondert erwähnt, jedoch stets mit umfasst.

Die reaktiven Aminogruppen können primäre oder sekundäre Aminogruppen sein. Mindestens eine der reaktiven Aminogruppen des Peptids ist bevorzugt Bestandteil einer Diaminosäure. Bevorzugt umfasst das Peptid daher mindestens eine Diaminosäure, bevorzugt mindestens 2, 3, 4, 5 oder mehr Diaminosäuren. Es können Lysin-haltige Peptide (bzw. Oligopeptide oder Eiweiße) eingesetzt werden.

Auch andere Aminosäuren, wie Arginin, Asparagin, Glutamin oder Histidin, tragen reaktive Aminogruppen, die mit den Oligolactonen reagieren können.

Die reaktiven Aminogruppen, insbesondere die durch die Diaminosäure bereitgestellten Aminogruppen, sind für die Vernetzungsreaktion zwischen Peptid und Oligolacton besonders geeignet. Auch Hydroxylgruppen in dem Peptid können zu der Vernetzungsreaktion beitragen. Bevorzugt umfasst das Peptid daher mindestens eine Aminosäure mit einer Hydroxylgruppe, also insbesondere Serin, Threonin oder Tyrosin. Auch Hydroxylysin oder polyphenolische Aminosäurebausteine, wie sie in den MAPs vorliegen, können in den erfindungsgemäß eingesetzten Peptiden vorkommen. Ein Vorteil der vorliegenden Erfindung ist jedoch, dass die Anwesenheit dieser spezifischen Aminosäurebausteine und damit die Verwendung von MAPs nicht notwendig ist. Die verwendeten Peptide können daher z.B. ohne weiteres rekombinant hergestellt werden.

Die durch Reaktion der Oligolactone mit dem Peptid entstehende Vernetzung hängt wesentlich davon ab, wie hoch der Anteil der zur Verfügung stehenden reaktiven Gruppen in dem Peptid ist.

Besonders gute Klebeeigenschaften können ab einem molaren Anteil an Aminosäuren des Peptids, die eine freie Aminogruppe (z.B. Diaminosäuren wie Lysin) und/oder Hydroxylgruppe tragen, von mindestens 10 % erreicht werden. Bevorzugt liegt der Anteil dieser Aminosäuren jedoch höher, bei mindestens 20%, mindestens 30%, mindestens 40 oder mindestens 50% oder sogar bei mindestens 80 bis 100%. Diese Kriterien werden von natürlich vorkommenden Peptiden und Proteinen erfüllt. Besonders geeignet sind z.B. MAPs.

Es können jedoch auch besonders geeignete kürzere, leicht künstlich herstellbare Peptide eingesetzt werden. In einer besonders bevorzugten Ausführungsform der Erfindung sind etwa 50% der Aminosäuren des Peptids Lysin und/oder etwa 50% der Aminosäuren des Peptids Tyrosin. Diese können z.B. als sich wiederholende Dipeptid-Einheit angeordnet sein. Etwa 50% bedeutet 40-60%. Auch eine andere Abfolge oder die Aufnahme weiterer Aminosäuren, insbesondere von Arginin, Asparagin, Glutamin oder Histidin (anstelle von Lysin oder zusätzlich), Serin oder Threonin (anstelle von Tyrosin oder zusätzlich) ist ohne weiteres möglich. Eine Länge des Peptids von 10-20 Aminosäuren ist besonders bevorzugt. Hervorragende Ergebnisse konnten etwa mit Peptiden von 10-20 Aminosäuren Länge, die aus sich wiederholenden Dipeptideinheiten von Lysin und Tyrosin bestanden ([Lys-Tyr]ₙ oder [Tyr-Lys]ₙ, n=5 bis n=10), erzielt werden.

Das Präparat enthält bevorzugt bis 40 Gew-% zusätzliches Peptid, insbesondere 10-30 Gew-% oder 20 Gew-%.

In einer weiteren Ausführungsform kann ferner zusätzlich Kettenverlängerer zur Bulkpolymerisation (ausgewählt aus einer Gruppe umfassend Diole, Diamine, Oligolactone wie EOL, EOG, GOL, GOLG, POL) in der erfindungsgemäßen Kombination enthalten sein. Die Anwesenheit eines solchen zusätzlichen Kettenverlängerers ist jedoch nicht zwingend notwendig.

Die im Rahmen der Erfindung verwendete Calcium-haltige Substanz ist bevorzugt Calciumphosphat, beispielsweise Tricalciumorthophosphat und/oder Hydroxylapatit. Bevorzugt umfasst das Präparat 20-90 Gew-% Calcium-haltige Substanz, insbesondere 40-80 Gew-% Calcium-haltige Substanz, 50-70 Gew-% Calcium-haltige Substanz oder 60-65 Gew-% Calcium-haltige Substanz.

Es kann auch eine Kombination mit einem geringeren Anteil an Calcium-haltiger Substanz verwendet werden, dies erreicht jedoch nicht die Härte, die zur Verwendung als "flüssiger Knochen" bzw. Knochenzement von besonderem Vorteil ist.

Bevorzugt sind in der Kombination Kollagen und/oder zusätzliches Peptid und Calcium-haltige Substanz bereits gemischt, sie können jedoch auch noch einzeln vorliegen.

Das Vernetzungsmittel umfasst ein Molekül, das zwischen dem Kollagen Brücken bilden und die Mischung damit stabilisieren und verfestigen kann. Dies ist ein reaktionsfähiges endständig funktionalisiertes Oligolacton.

Als Oligolactone werden im Rahmen der Erfindung "innere Ester" von Hydroxycarbonsäuren und somit im erweiterten Sinne auch Oligoglykolide, Oligolactide und deren Copolymere bezeichnet, die, wie im Stand der Technik bekannt, durch ringöffnende Polymerisation hergestellt werden können. Unter endständig funktionalisierten Oligolactonen werden Polymere von Hydroxycarbonsäuren mit einem zentralen mindestens zweiwertigen Alkohol verstanden, wobei die Hydroxylgruppen des Alkohols mit Lactonen bzw. Lactiden oder Glykoliden durch Ringöffnung verestert sind und wobei die freien Enden der Polyester reaktionsfähige funktionelle Gruppen tragen. Ein Beispiel ist in Reaktionsschema 1 dargestellt.

Bevorzugt weist das Oligolacton endständige Isocyanatgruppen auf. Auch eine Funktionalisierung mit Aldehyd- oder Epoxidgruppen ist möglich, und das erhaltene Produkt kann in der erfindungsgemäßen Kombination eine Klebewirkung erzielen. Es stellte sich überraschenderweise heraus, dass durch Funktionalisierung der Oligolactone mit Isocyanat sowohl eine erhöhte Festigkeit des Polymerisats als auch eine größere Haftfestigkeit erreicht wurde.

Bevorzugt ist das Oligolacton ein Ethylenglycol-Oligolactid (EOL), wie es in Reaktionsschema 1 dargestellt ist. In einer Ausführungsform der Erfindung ist n = 1 (bei Umsetzung von 1 Teil Ethylenglykol mit 2 Teilen Lactid, d. h. 1/2 ist M = 350,3 g/mol), n kann jedoch auch 2, 3, 4 oder 5 sein. Mit zunehmender Anzahl der Einheiten steigt die Viskosität des Oligolactons und der im Rahmen einer Reaktion mit den Peptiden erhaltenen Kleber an. Eine optimale Handhabbarkeit wurde mit Oligolactonen (n=1) erzielt, da dadurch eine gute Verarbeitung bei einem geringen Lösungsmittelanteil gewährleistet war. Außerdem wurden weitere Ethylenglykol-Derivate wie Ethylenglycololigoglycolid (EOG 1/2, M = 294,2 g/mol) mit endständigen Isocyanatgruppen versehen. Auch mit diesen Derivaten wurden gute Klebeeigenschaften erhalten.

Analog ist auch die Verwendung weiterer Oligolactone, etwa auf der Basis von Polymerisationsprodukten von Glycerol und Pentaerythrit möglich. So entstehen beispielsweise bei der Umsetzung von Pentaerythritooligolactid (POL 1/4, M = 712,6 g/mol), Glycerololigolactid (GOL 1/0,5, M= 164,1 g/mol) und Glycerololigolactid-co-glycolid (GOLG 1/1/3, M = 584,4 g/mol) mit Hexamethylendiisocyanat (HDI) folgende Produkte mit endständigen Isocyanatgruppen: POL-NCO, EOG-NCO und GOLG-NCO.

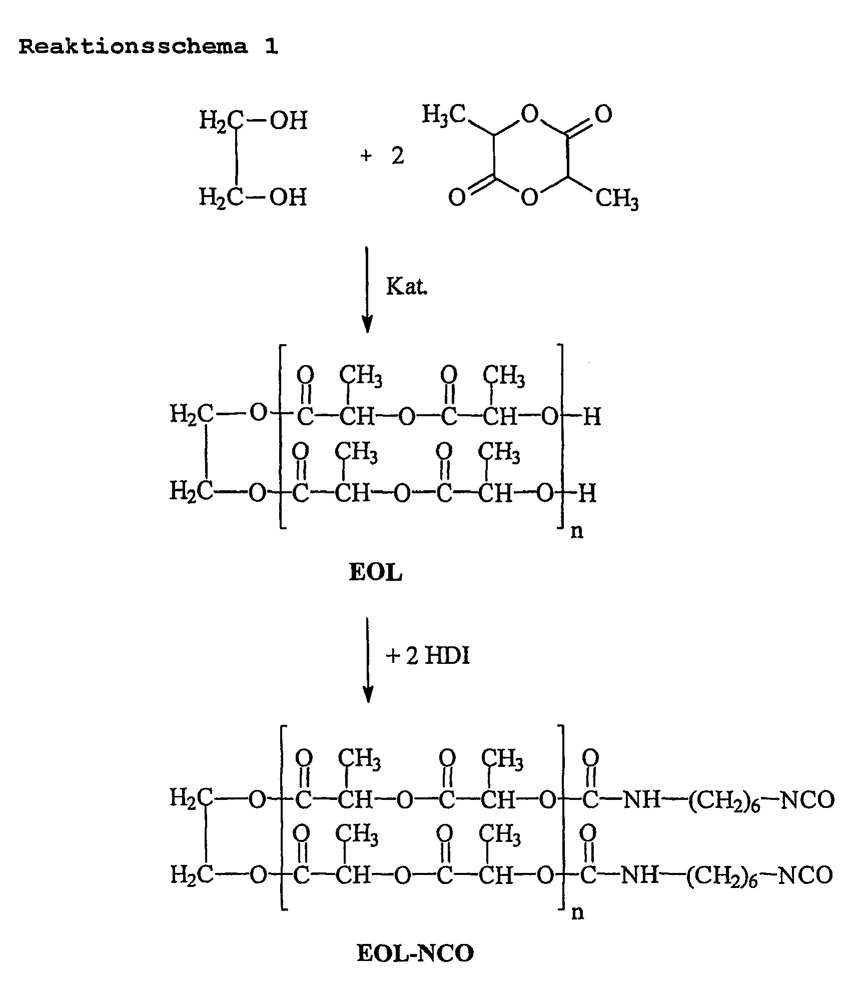

Selbstverständlich können auch mehrere verschiedene Oligolactone eingesetzt werden. Diese Variante ist in der Folge nicht gesondert erwähnt, jedoch stets mit umfasst.

Als Katalysator für die Herstellung der Oligolactone können organische Metallverbindungen, etwa Zink- oder Zinnverbindungen eingesetzt werden (H. R. Kricheldorf, H. R.,Kreiser-Saunders, I., Boettcher, C.:" Polylactones: 31. Sn(II)octoate-initiated polymerization of L-lactide: a mechanistic study",in: Polymer Vol. 36 No. 6, pp. 1253-1259, 1995; Kreiser-Saunders, I., Kricheldorf, H. R.:". Polylactones: 39a.

Zn lactate-catalyzed copolymerisation of L-lactide with glycolide or ε-caprolactone", in: Macromol. Chem. Phys. 199, 1081-1087, 1998; Kricheldorf, H. R., Kreiser-Saunders, I., Damrau, D. O:" Resorbable Initiators for Polymerization of Lactones", in: Macromol. Symp. 144, 269-276, 1999; Kricheldorf, H. R., Kreiser-Saunders, I.:"Polylactides-Synthesis, Characterization and Medical Application", in: 103, 85-102, 1996).

Beispiele sind Zink- bzw. Zinn-(II)-salze von organischen Carbonsäuren, z.B. Zink-(II)-octoat, Zink-(II)-ethyl-hexoat, Zinn-(II)acetat, Zinn-(II)-octoat, Zinn-(II)-ethylhexoat und Zinn-(II)-laurat, und Dialkylzinn(IV)-salze von organischen Carbonsäuren, z.B. Dibutyl-zinndiacetat, Dibutylzinn-dilaurat, Dibutylzinn-maleiat und Dioctylzinn-diacetat. Weiterhin können auch Eisen(III)-salze verwendet werden, wie z.B. Eisen(III)-chlorid.

Um die Bioverträglichkeit zu erhöhen, werden die Katalysatoren dieser Reaktion vor der Verwendung der Oligolactone bevorzugt entfernt, so dass sie in Mengen von unter 0,1%, bevorzugt von unter 0,01% vorliegen. Dazu sind im Stand der Technik Verfahren bekannt (EP1497340, EP1221454).

Alternativ oder zusätzlich werden, vor allem zur Herstellung von Medizinprodukten, die besser biologisch verträglichen Zinkverbindungen oder Eisenverbindungen eingesetzt.

Die Oligolactone können mit Diisocyanaten, z.B. mit Hexamethylendiisocyanat umgesetzt werden, um Oligolactone mit endständigen Isocyanatgruppen herzustellen. Bevorzugt werden für die Umsetzung aliphatische Isocyanate verwendet, da sich aus aromatischen Diisocyanaten kanzerogene Diamine bilden können. Bei einer stöchiometrischen Umsetzung ist eine Aufreinigung nicht nötig. Gegebenenfalls kann jedoch eine Aufreinigung z.B. über Destillation durchgeführt werden.

Vorzugsweise umfasst das erfindungsgemäße medizinische Präparat 0,1 bis 40 Gew-% Vernetzungsmittel, insbesondere 1 bis 30 Gew-%, 2 bis 20 Gew-% oder 5 bis 10 Gew-% Vernetzungsmittel. Dabei sind bevorzugt mindestens 70 Gew-%, mindestens 80 Gew-%, mindestens 90 oder mindestens 95 oder 99% Gew-% des Vernetzungsmittels endständig funktionalisiertes Oligolacton.

Sofern eine schnelle Aushärtung gewünscht ist, umfasst das Vernetzungsmittel einen Katalysator der Vernetzungsreaktion. Der Katalysator ist in der Lage, eine Vernetzung zwischen den Kollagenmolekülen und/oder weiteren Peptiden und Molekülen mit reaktiven funktionellen Gruppen herbeizuführen. Diese Vernetzung kann eine direkte Vernetzung sein, z.B. über Disulfidbrücken, oder eine indirekte Vernetzung über ein Brückenmolekül. In diesem Fall vermittelt der Katalysator die Reaktion zwischen Kollagen und/oder zusätzlichem Peptid und Oligolacton.

Der Anteil an Katalysator am Vernetzungsmittels beträgt 0-20 Gew.-%, bevorzugt 0,05-10 Gew.-%, 0,1-8 Gew.-%, 1-6 Gew.-% oder 3-5 Gew.-%.

Katalysatoren beschleunigen die Reaktionsgeschwindigkeit einer chemischen Reaktion, ohne dabei selbst verbraucht zu werden. Eine Beschleunigung (etwa um den Faktor 10-100) ist, abhängig auch von den Ausgangsstoffen und dem genauen Einsatzgebiet, oft sinnvoll. Es kann jedoch auch eine langsamere Reaktionsgeschwindigkeit bevorzugt sein, so dass z.B. eine längere Zeit für die Verarbeitung zur Verfügung steht. Insbesondere beim Einsatz von zusätzlichen Oligopeptiden mit einem hohen Anteil (mindestens 30%, bevorzugt mindestens 50 %) von Aminosäuren mit reaktiven Aminogruppen, vorzugsweise Diaminosäuren, ist die Reaktionsgeschwindigkeit auch ohne Katalysator so hoch, dass dieser für eine schnelle Aushärtung nicht notwendig ist.

Mit Katalysator wird mit diesen Peptiden eine besonders schnelle Aushärtung erreicht.

Mit dem erfindungsgemäßen Präparat kann bevorzugt eine feste Aushärtung im Zeitrahmen von 30 Sekunden bis 15 Minuten, besser 1 bis 5 Minuten erfolgen. In dieser Zeit muss jedoch nicht eine vollständige Reaktion aller Komponenten, d. h. eine vollständige Bulkpolymerisation, erfolgt sein, sondern es reicht aus, wenn eine Festigkeit erreicht wird, bei der die verklebten Substrate fixiert sind. Die Reaktion läuft mit Katalysator in einem Zeitraum von 3-60 Minuten, vorzugsweise 3-10 Minuten im wesentlichen vollständig ab, ohne Katalysator beträgt der Zeitraum für einen im wesentlichen vollständigen Ablauf der Reaktion 30 Minuten bis zu mehreren Tagen, vorzugsweise 30-120 Minuten.

Als Katalysator kann etwa ein basisches Amin, ein Amidin, vorteilhafterweise 2,3-Dimethyl-3,4,5,6-tetrahydropyrimidin, ein tertiäres Amin, vorteilhafterweise Triethylamin, Tributylamin, Dimethylbenzylamin, N-Methyl-, N-Ethyl-,N-Cyclohexylmorpholin, N,N,N',N'Tetramethyl-ethylendiamin, N,N,N',N'-Diaminoethylether, Bis-(dimethylaminopropyl)harnstoff, Dimethylpiperazin, 1,2-Dimethylimidazol, 1-Aza-bicyclo-(3,3,0)-octan und vorzugswiese 1,4-Diaza-bicyclo-(2,2,2)-octan und/oder ein Alkanolamin, wie Triethanolamin, Triisopropanolamin, N-Methyl- und N-Ethyl-diethanolamin und Di-methylethanolamin, vorzugsweise 1,4-Diaza[2.2.2]bicyclooctan (Dabco), eingesetzt werden. Bevorzugt ist der Katalysator 1,4-Diaza[2.2.2]bicyclooctan (Dabco).

In einer besonders bevorzugten Ausführungsform ist in der Kombination Kollagen, eine Calcium-haltige Substanz, insbesondere Hydroxylapatit, ein reaktionsfähiges endständig funktionalisiertes Oligolacton, insbesondere Ethylenglycololigolactid mit endständigen Isocyanatgruppen und der Katalysator 1,4-Diaza[2.2.2]bicyclooctan (Dabco) enthalten, unter Zusatz eines weitere Oligopeptid oder protein, das mindestens zwei reaktive Aminogruppen und/oder Hydroxylgruppen umfasst.

Es können Wirkstoffe in der Kombination enthalten sein, die, soweit sie in der Lage sind, mit den Komponenten zu reagieren, am Ort des Einsatzes fixiert werden können und dort dauerhaft wirken. Denkbar sind z.B. Antibiotika, weitere antimikrobielle Wirkstoffe und/oder das Immunsystem hemmende oder fördernde Substanzen und/oder Botenstoffe, die den Aufbau von Knochensubstanz fördern und/oder ihren Abbau hemmen. Es können weitere Zusatz- und Hilfsstoffe enthalten sein, z.B. Füllmittel wie Albumin, Hyaluronsäure oder ähnliches. Auch Zusatzstoffe wie Thixotropiemittel, z.B. nanodisperse Calciumphosphate (z.B. beta-Tricalciumphosphat (beta-TCP)) oder nanodisperse Kieselsäuren können zur Anpassung von Viskosität und Fließfähigkeit verwendet werden.

In einer Ausführungsform der Erfindung liegen eine oder mehrere der Komponenten der Kombination getrennt von einander vor. Es können z.B. alle Komponenten getrennt vorliegen. Es kann jedoch auch eine Vormischung von Komponenten erfolgen, die die Verwendung vorbereitet, beispielsweise können Kollagen/Peptid und Calcium-haltige Substanz bereits gemischt sein. Auch eine vorbereitete Mischung einer oder mehrerer dieser Komponenten, die den Katalysator umfasst, kann im Allgemeinen noch gut gelagert werden. Eine Lagerung bei ca. 4°C ist empfehlenswert. Vorteilhafterweise werden aber das Oligolacton und Kollagen und/oder zusätzliches Biopolymer/Peptid erst dann gemischt, wenn die Reaktion zwischen beiden erwünscht ist. Wenn jedoch der Katalysator erst direkt vor der Anwendung hinzugefügt wird, ist auch eine Mischung, die sowohl Kollagen bzw. Biopolymer als auch Oligolacton umfasst, noch lagerfähig.

Eine oder mehrere Komponenten der Kombination können - gemeinsam oder getrennt - in einem oder mehreren Lösungsmitteln aufgenommen sein. Das Lösungsmittel ist bevorzugt nicht toxisch und in geringen Mengen biologisch verträglich. Es kann ein wässriges Lösungsmittel sein, z.B. Phosphatpuffer, insbesondere ein Calciumphosphatpuffer, oder ein organisches Lösungsmittel wie DMSO, insbesondere bei Verwendung von mit Isocyanat funktionalisierten Oligolactonen, oder eine Mischung daraus. In einer Ausführungsform wird als Lösungsmittel eine Mischung von DMSO und Wasser (oder Puffer) verwendet, wobei der Anteil von Wasser 5-20% beträgt, bevorzugt 8-12% oder 10%. Insbesondere wird das Kollagen/Peptid (und gegebenenfalls der Katalysator) in dem wässrigen bzw. wasserhaltigen Lösungsmittel gelöst und das Oligolacton in DMSO.

In einer Ausführungsform ist das Präparat bereits in einer Zweikomponentenspritze, bevorzugt bereits mit angesetztem Mischextruder, zur Anwendung vorbereitet, wobei die eine Komponente Kollagen/Peptid und gegebenenfalls den Katalysator umfasst und die andere Komponente das Oligolacton. Dies erlaubt eine besonders exakte Dosierung und einfache Handhabung. Bevorzugt ist eine Doppelkammerspritze etwa vom Typ Mixpac (Mixpac Systems AG, Rotkreuz, Schweiz).

Alternativ kann die Kombination oder ihre Bestandteile direkt vor der Verwendung in einem Lösungsmittel aufgenommen werden.

Bevorzugt liegen die Bestandteile der Kombination schon in einem Mengenverhältnis vor, das für ihre Verwendung angemessen ist und ein umständliches Dosieren der Zutaten erspart. Bevorzugt sind die Komponenten steril verpackt.

Eine Sterilisierung des erfindungsgemäßen Produkts bzw. seiner einzelnen Komponenten kann vorteilhafterweise ohne Strukturveränderung erreicht werden, z.B. über Sterilfiltrierung von Lösungen. Bevorzugt ist jedoch eine Sterilisierung über GammaStrahlung, da diese bereits verpackt erfolgen kann und somit keine aseptische Abfüllung nötig ist. Es konnte gezeigt werden, dass bei Gamma-Sterilisation die Struktur der funktionalisierten Lactone erhalten bleibt.

Bevorzugt ist die erfindungsgemäße Kombination ein Medizinprodukt, das zur Behandlung der Osteoporose und/oder zur Prophylaxe und Behandlung von Knochenbrüchen geeignet ist. Eine Klassifizierung als pharmazeutisches Präparat ist jedoch, abhängig von nationalem Recht, ebenfalls möglich. Die Begriffe Medizinprodukt und pharmazeutisches Präparat sind für die Zwecke der Beschreibung der Erfindung austauschbar.

Gegenstand der Erfindung ist auch ein Produkt, in dem die oben genannten Komponenten miteinander vermischt sind und das Kollagen, ggf. unter Einbeziehung weiterer Biopolymere, mit einander vernetzt ist. Bevorzugt ist das Kollagen, gegebenenfalls unter Einbeziehung weiterer reaktiver Substrate, über das im Rahmen der Erfindung verwendete Oligolacton miteinander vernetzt. Dabei kann, muss aber keine vollständige Reaktion erfolgt sein, solange eine ausreichende Festigkeit des ausgehärteten Produkts erreicht wird.

Ein solches Produkt kann in Form eines künstlichen Knochens, Knochenteils, oder Implantats, insbesondere zur Knochenbruchstabilisierung, oder in Form einer Implantat-Beschichtung vorliegen. Insbesondere ist ein solches Implantat ein Nagel, eine Platte, eine Schraube, ein Stift, eine Prothese, eine Hüftpfanne, ein Wirbelkörperersatz oder ein "cage" (Käfig als Wirbelkörperersatz).

Die derartig hergestellten Produkte ähneln in ihrer Konsistenz dem menschlichen oder tierischen Knochen und sind deshalb, und wegen ihrer guten biologischen Verträglichkeit, hervorragend zur Implantation geeignet und können anstelle der bislang verwendeten Strahl- oder Titanimplantate verwendet oder mit diesen kombiniert werden. Unter anderem ist die Kombination geeignet zur Beschichtung von Implantaten aus anderen Materialien. Zu diesem Zweck werden auch Plasma-Beschichtungsverfahren in Betracht gezogen.

Gegenstand der Erfindung ist auch die Verwendung der geschilderten Kombination in Kombination mit Implantaten aus anderen Materialien, z.B. Titan. Die erfindungsgemäße Kombination wird in diesem Fall anstelle von herkömmlichem Knochenzement verwendet und fixiert das Implantat.

Die Erfindung betrifft ferner ein Medizinprodukt, das die oben geschilderten Komponenten umfasst und zur Behandlung von Osteoporose und/oder zur Prophylaxe und Behandlung von Knochenbrüchen geeignet is, sowie die Verwendung der oben beschriebenen Kombination zur Behandlung von Osteoporose und/oder zur Prophylaxe und Behandlung von Knochenbrüchen. Insbesondere sollen osteoporotische Knochen bei drohender Fraktur prophylaktisch stabilisiert werden. Auch bei reduzierter Knochendichte oder anderen Erkrankungen, die mit einem erhöhten Risiko an Frakturen einhergehen, kann eine Verwendung jedoch sinnvoll sein, z.B. bei Glasknochenkrankheit.

Zur Verwendung werden die Komponenten der Kombination miteinander vermischt und mit Knochen in Kontakt gebracht. Bevorzugt werden die Komponenten der Kombination mit einem Lösungsmittel gemischt und in flüssiger oder breiiger Form in den Knochen eingespritzt. Dort härtet das Produkt aus.

Durch die Erfindung wird damit erstmals ein "flüssiger Knochen" bereitgestellt, der nicht auf Basis von künstlichen Polymeren, sondern auf Basis von vernetztem Kollagen zubereitet ist. Die erfindungsgemäße Kombination enthält somit in einer bevorzugten Ausführungsform kein PMMA oder andere Acrylate.

Bei der Anwendung wird bevorzugt eine minimalinvasive Technik verwendet. Durch die Vernetzung des Kollagens und Verbindung mit der Calcium-haltigen Substanz sowie dem umgebenden Knochenmaterial wird der Knochen stabilisiert.

Die Kombination kann nicht nur bei frakturgefährdeten Knochen im Rahmen einer Frakturprophylaxe, sondern auch bei bereits erfolgter Fraktur im Rahmen der operativen Versorgung derselben genutzt werden, und zwar sowohl zur Behandlung der Fraktur als auch zur Prophylaxe einer Fraktur an einer anderen, insbesondere benachbarten Stelle, z.B. des benachbarten Wirbels oder der benachbarten Wirbel. Dabei wird der Vorteil der bereits erfolgten Betäubung genutzt. Die erfindungsgemäße Kombination kann auch bei klassischer Vertebroplastie und/oder Kyphoplastie verwendet werden.

Gegenstand der Erfindung ist auch die Verwendung von Kollagen, zusätzlichem Oligopeptid oder protein, einer Calcium-haltigen Substanz und einem Vernetzungsmittel zur Herstellung eines Medizinprodukts oder pharmazeutischen Präparats zur Behandlung und/oder Prophylaxe von Osteoporose und/oder Prophylaxe und Behandlung von Knochenbrüchen, wobei das Vernetzungsmittel ein reaktionsfähiges endständig funktionalisiertes Oligolacton und bevorzugt einen Katalysator umfasst. Eine weitere Anwendungsmöglichkeit findet sich im Bereich der Zahnmedizin, z.B. bei der Klebung von Inlays und Kronen oder bei Aufbaufüllungen. Auch die elastische Fixierung von Wurzelstiften mit der erfindungsgemäßen Kombination anstelle von klassischem "Sealer" auf Basis von Methylmethacrylaten oder Glasionomer-Zementen ist möglich.

Im Hinblick auf die bevorzugt verwendeten Komponenten wird zur Vermeidung von Wiederholungen auf die obigen Ausführungen zur erfindungsgemäßen Kombination Bezug genommen, die hier gleichermaßen gelten.

Ferner stellt die Erfindung ein *in vitro*-Verfahren zur Herstellung künstlicher Knochen, Knochenteile, Zähne, Implantate oder Implantatbeschichtungen zur Verfügung, bei dem man die oben beschriebenen Komponenten der Kombination miteinander in Kontakt und in die gewünschte Form bringt. Insbesondere werden im Rahmen dieses Verfahrens die Komponenten miteinander gemischt, in eine Form gefüllt und härten nach Vernetzung aus. Alternativ kann eine Kollagen-haltige Matrix der gewünschten Form mit den anderen Komponenten in Kontakt gebracht werden.

Gegenstand der Erfindung ist auch ein künstlicher Knochen, ein künstliches Knochenteil, ein Implantat, insbesondere ein Implantat zur Knochenbruchstabilisierung, oder eine Implantat-Beschichtung, die nach dem erfindungsgemäßen, oben beschriebenen Verfahren erhalten werden kann. Auch künstliche Zähne, Kronen oder Inlays werden im Rahmen der Erfindung als Implantate verstanden.

Ein besonderer Vorteil der vorliegenden Erfindung ist, dass die nach der Vernetzung erhaltene künstliche Knochensubstanz biologisch gut verträglich ist. Die anfängliche Härtungsphase, verbunden mit hohen Klebeigenschaften, und die langsame Resorption im Körper bei fortgeschrittenen Heilungsprozessen, ist ein weiterer Vorteil.

Die Erfindung wird nachfolgend anhand von Beispielen erläutert. Die folgenden Beispiele dienen dazu, den Gegenstand der Erfindung beispielhaft zu illustrieren, die Erfindung ist jedoch nicht auf die genannten Beispiele begrenzt.

### BEISPIEL

### Beispiel 1: Flüssiger Knochen mit Ethylenglycololigolactid mit endständigen Isocyanatgruppen

Ein besonders stabiler Kleber zur Stabilisierung von Knochen wurde mit Ethylenglycololigolactid mit endständigen Isocyanatgruppen (EOL-NCO) als Brückenmolekül hergestellt.

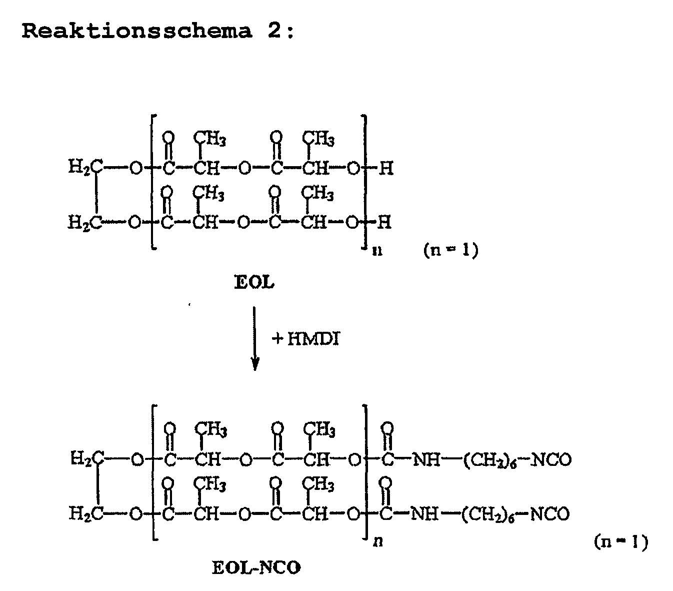

Ethylenglycololigolactid (EOL) wurde, entsprechend Reaktionsschema 2, mit Hexamethylendiisocyanat (HMDI) umgesetzt. Produkt der Reaktion war EOL-NCO (Ethylenglycololigolactid mit endständigen Isocyanatgruppen), das in dem Kleber als Brückenmolekül eingesetzt wurde.

Zur Aushärtung des Klebers wird EOL-NCO in Dimethylsulfoxid (DMSO) und eine Mischung aus Oligopeptid (einem Polymer aus Tyrosin und Lysin, siehe Beispiel 2) und 1,4-Diaza[2.2.2]bicyclooctan (Dabco) als Katalysator, ebenfalls in DMSO als Lösungsmittel, miteinander und mit einer Mischung aus Kollagen und einer Calcium-haltigen Substanz in Kontakt gebracht.

### Beispiel 2:

Die in diesem Versuch eingesetzten Komponenten waren Kollagen, Oligopeptid 1 (Aminosäuresequenz aus Lysin und Tyrosin, wobei bevorzugt ein Oligopeptid n=5 bis n=10 verwendet wurde, Ethylenglycololigolactid mit endständigen Isocyanatgruppen (EOL-NCO) und 1,4-Diaza[2.2.2]bicyclooctan (Dabco) als Katalysator, DMSO als Lösungsmittel, sowie Hydroxylapatit.

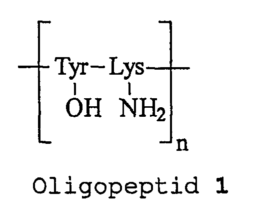

### Herstellung von EOL-NCO:

Ethylenglycol-Oligolactid (1/2, d.h. Umsetzung von 1 Teil Ethylenglykol mit 2 Teilen Lactid bei der ringöffnenden Polymerisation, n=1) wurde mit Hexamethylendiisocyanat (HDI) umgesetzt (siehe Reaktionsschema 2) und es resultierte aus der Reaktion EOL-NCO (Ethylenglycol-Oligolactid mit endständigen Isocyanatgruppen), das in den weiteren beschriebenen Versuchen verwendet wurde.

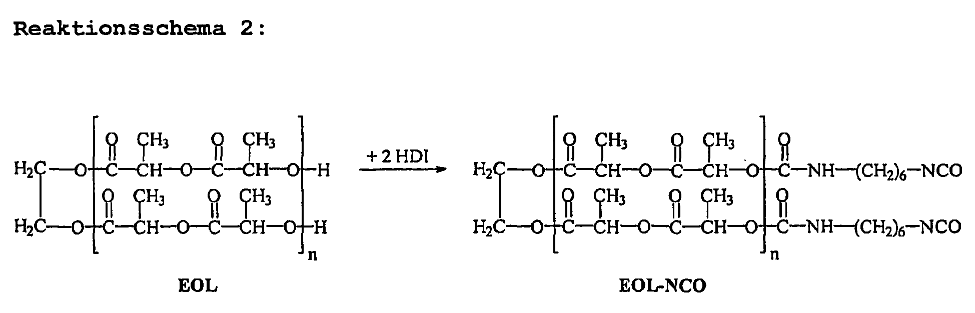

### Mischung der Komponenten:

Die Aushärtung wurde mit dem System EOL-NCO in Dimethylsulfoxid (DMSO) (Komponente 1) und Hydroxylapatit/Peptide/1,4-Diaza[2.2.2]bicyclooctan (Dabco) als Katalysator in DMSO (Komponente 2) durchgeführt (Reaktionsschema 3).

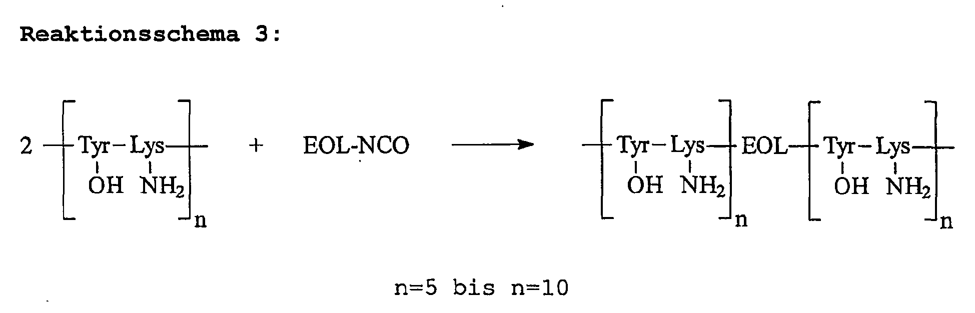

Die Struktur des Polymerisats ist nur schematisch dargestellt, wobei die Vernetzung nicht anhand von Kollagen, sondern anhand des zusätzlichen Peptids gezeigt ist. Dabei wird davon ausgegangen, dass die Reaktion des EOL-NCO mit den primären Aminogruppen und Hydroxylgruppen des Peptids erfolgt.

Es wurde folgendes Mischungsverhältnis geprüft: EOL-NCO/Peptide 4,7/1 (w/w) und EOL-NCO/Dabco 12,5/1 (w/w).

### Beispiel 3: Fixierung von Implantaten

Prothesen der Hüfte, des Knies und der Schulter werden mit einer festen, tragenden Komponente in den hohlen Knochen eingebolzt, nachdem das Knochengewebe reseziert wurde und somit eine Öffnung im Knochen besteht. Dabei ist eine Verklemmung möglich, die ggf. keine zusätzlichen Fixierhilfen braucht. Falls der Formschluss nicht erzielt werden kann, oder eine sofortige Belastung des Implantates gewünscht wird, werden Prothesen im hohlen Knochen mit einem Zementmantel aus dem erfindungsgemäßen Präparat versehen.

In diesem Falle wird nach Aufraffeln des Markraumes, bei dem das Knochenmark größtenteils entfernt wird, Kollagen, gegebenenfalls zusätzliches Peptid, Calcium-haltige Substanz und Vernetzungsmittel vermengt und vom Operateur in den Knochen eingespritzt, genauer gesagt in den hohlen Markraum des Knochens. In noch flüssigem Zustand wird dann die Prothese eingebracht, die dann durch den Prozess des Aushärtens des Knochenklebers fixiert wird. Diesen 2-10 Minuten dauernden Prozess wartet der Operateur ab, bis er mit der Operation fortfährt.

### Beispiel 4: Prophylaxe von Frakturen

Um brüchigen Knochen ohne Fraktur zu stabilisieren, wird dieser - ähnlich der Vertebroplastie - mit einem Bohrer oder einer scharfen Nadel angebohrt. Die Lage der Nadel wird mit einem Röntgenbild kontrolliert. Dann wird der flüssige Knochen, wie in Beispiel 1 oder 2 angerührt und in flüssiger Form in den Knochen appliziert. Gegebenenfalls muss auch hier der Markraum präpariert werden, dies sollte aber nicht das primäre Ziel sein. Vielmehr sollte sich nun der breiige Knochen im Markraum dort verteilen, wo wenig Knochensubstanz ist und dort aushärten. Auch das Verteilungsergebnis wird mit Röntgen kontrolliert. Bevor der Zement aushärten kann, muss die eingebrachte Nadel entfernt werden. Die Operation kann in Lokalanästhesie oder in Allgemeinnarkose durchgeführt werden. Sterile Bedingungen sind während jeder Operation zu beachten.

## Patentansprüche

1. Kombination zur Behandlung von Osteoporose und/oder Prophylaxe und Behandlung von Knochenbrüchen oder zur Fixierung von Implantaten, die
- Kollagen,
- zusätzliches Oligopeptid oder Protein, das mindestens zwei reaktive Aminogruppen und/oder Hydroxylgruppen umfasst,
- eine Calcium-haltige Substanz und
- ein Vernetzungsmittel umfasst, wobei das Vernetzungsmittel ein reaktionsfähiges endständig funktionalisiertes Oligolacton umfasst.

2. Kombination nach den Anspruch 1, **dadurch gekennzeichnet, dass** das Oligolacton ein Ethylenglycol-Oligolacton ist, welches bevorzugt endständige Isocyanatgruppen aufweist.

3. Kombination nach den Ansprüchen 1 bis 2, **dadurch gekennzeichnet, dass** das Vernetzungsmittel ferner einen Katalysator der Vernetzungsreaktion umfasst.

4. Kombination nach Anspruch 3, **dadurch gekennzeichnet, dass** der Katalysator ein stark basisches Amin, Amidin, tertiäres Amin und/oder Alkanolamin umfasst.

5. Kombination nach nach den Ansprüchen 1 bis 4, **dadurch gekennzeichnet, dass** 50% der Aminosäuren des Oligopeptids oder Proteins Lysin und/oder 50% der Aminosäuren des Oligopeptids oder Proteins Tyrosin sind.

6. Kombination nach den Ansprüchen 1 bis 5, **dadurch gekennzeichnet, dass** das Oligopeptid ein Oligopeptid mit 10-20 Aminosäuren Länge ist, bei dem 50% der Aminosäuren Lysin und/oder etwa 50% der Aminosäuren Tyrosin sind, dass die Calcium-haltige Substanz Hydroxylapatit ist und dass das Vernetzungsmittel ein Ethylenglycol-Oligolactid mit endständigen Isocyanatgruppen und 1,4-Diaza[2.2.2]bicyclooctan (Dabco) umfasst.

7. Kombination nach den Ansprüchen 1 bis 6, **dadurch gekennzeichnet, dass** sie Kollagen in einem Anteil von 10-70 Gew-%, Calcium-haltige Substanz in einem Anteil von 20-90 Gew-%, Vernetzungsmittel in einem Anteil von 0,05 bis 40 Gew-%, und das zusätzliche Oligopeptid oder Protein in einem Anteil von 0-40 Gew-% enthält.

8. Kombination nach den Ansprüchen 1 bis 7, **dadurch gekennzeichnet, dass** eine oder mehrere der Komponenten getrennt von einander vorliegen, wobei optional eine oder mehrere Komponenten in einem Lösungsmittel aufgenommen sind.

9. Kombination nach den Ansprüchen 1 bis 7, **dadurch gekennzeichnet, dass** die Komponenten miteinander vermischt sind und das Kollagen vernetzt ist.

10. Kombination nach Anspruch 9, **dadurch gekennzeichnet, dass** sie in Form eines künstlichen Knochens, Knochenteils, Implantats oder in Form einer Implantat-Beschichtung vorliegt, wobei das Implantat optional ein Nagel, eine Platte, eine Schraube, ein Stift, eine Prothese, eine Hüftpfanne, ein "cage" oder ein Wirbelkörperersatz ist.

11. Medizinprodukt, **dadurch gekennzeichnet, dass** es die Kombination nach den Ansprüchen 1-10 umfasst.

12. Medizinprodukt nach Anspruch 11 zur Behandlung von Osteoporose und/oder Prophylaxe und Behandlung von Knochenbrüchen oder zur Fixierung von Implantaten.

13. Verwendung der Kombination nach Anspruch 8 zur Herstellung eines Medizinprodukts zur Behandlung von Osteoporose und/oder Prophylaxe und Behandlung von Knochenbrüchen oder zur Fixierung von Implantaten.

14. Verwendung nach Anspruch 13, **dadurch gekennzeichnet, dass** das Medizinprodukt dafür formuliert ist, dass man die Komponenten der Kombination miteinander vermischt und mit Knochen und/oder Implantat in Kontakt bringt.

15. Verfahren zur *in vitro* Herstellung künstlicher Knochen, Knochenteile, Implantate oder Implantatbeschichtungen, **dadurch gekennzeichnet, dass** man die Komponenten der Kombination nach Anspruch 8 miteinander in Kontakt und in die gewünschte Form bringt.

## Claims

1. A combination for the treatment of osteoporosis and/or prophylaxis and treatment of bone fractures or for the fixing of implants, comprising
- collagen,
- additional oligopeptide or protein which comprises at least two reactive amino groups and/or hydroxyl groups,
- a calcium-containing substance and
- a crosslinker, wherein the crosslinker comprises a reactive terminally functionalized oligolactone.

2. The combination of claim 1, wherein the oligolactone is an ethylene glycol oligolactone which preferably has terminal isocyanate groups.

3. The combination of claims 1 to 2, wherein the crosslinker further comprises a catalyst of the crosslinking reaction.

4. The combination of claim 3, wherein the catalyst comprises a strongly basic amine, amidine, tertiary amine and/or alkanolamine.

5. The combination of claims 1 to 4, wherein 50% of the amino acids of the oligopeptide or protein are lysine and/or 50% of the amino acids of the oligopeptide or protein are tyrosine.

6. The combination of claims 1 to 5, wherein the oligopeptide is an oligopeptide with a length of 10-20 amino acids, wherein 50% of amino acids are lysine and/or about 50% of amino acids are tyrosine, wherein the calcium-containing substance is hydroxylapatite, and wherein the crosslinker comprises an ethylene glycol oligolactide having terminal isocyanate groups and 1,4-diaza[2.2.2]bicyclooctane (Dabco).

7. The combination of claims 1 to 6, comprising collagen in a proportion of 10-70% by weight, calcium-containing substance in a proportion of 20-90% by weight, crosslinker in a proportion of 0.05 to 40% by weight, and the additional oligopeptide or protein in a proportion of 0-40% by weight.

8. The combination of claims 1 to 7, wherein one or more of the components are present separate from one another, wherein optionally one or more components are taken up in a solvent.

9. The combination of claims 1 to 7, wherein the components are mixed together, and the collagen is crosslinked.

10. The combination of claim 9, which is in the form of an artificial bone, bone part, implant or in the form of an implant coating, wherein the implant is optionally a nail, a plate, a screw, a pin, a prosthesis, a hip socket, a cage or a vertebra replacement.

11. A medical product, comprising the combination of claims 1 to 10.

12. The medical product of claim 11 for the treatment of osteoporosis and/or prophylaxis and treatment of bone fractures or for fixing implants.

13. The use of the combination of claim 8 for producing a medical device for the treatment of osteoporosis and/or prophylaxis and treatment of bone fractures or for fixing implants.

14. The use of claim 13, wherein the medical product is formulated for mixing the components of the combination together and bringing them into contact with bone and/or implant.

15. A method for the *in vitro* preparation of artificial bones, bone parts, implants or implant coatings, comprising bringing the components of the combination of claim 8 into contact with one another and bringing them into the desired shape.

## Revendications

1. Combinaison pour le traitement d'une ostéoporose et/ou la prophylaxie ou le traitement de fractures osseuses, ou pour la fixation d'implants, qui comprend:
- du collagène,
- un oligopeptide ou une protéine supplémentaire, qui comporte au moins deux groupes réactifs amino et/ou hydroxyle,
- une substance contenant du calcium,
- et un agent de réticulation, lequel agent de réticulation comprend une oligolactone réactive, fonctionnalisée en position terminale.

2. Combinaison conforme à la revendication 1, **caractérisée en ce que** l'oligolactone est une éthylèneglycol-oligolactone, qui est de préférence dotée de groupes isocyanate terminaux.

3. Combinaison conforme à la revendication 1 ou 2, **caractérisée en ce que** l'agent de réticulation comprend en outre un catalyseur de la réaction de réticulation.

4. Combinaison conforme à la revendication 3, **caractérisée en ce que** le catalyseur comprend une amine fortement basique, une amidine, une amine tertiaire et/ou une alcanolamine.

5. Combinaison conforme à l'une des revendications 1 à 4, **caractérisée en ce que** 50 % des résidus d'acides aminés de l'oligopeptide ou de la protéine sont des résidus de lysine et/ou 50% des résidus d'acides aminés de l'oligopeptide ou de la protéine sont des résidus de tyrosine.

6. Combinaison conforme à l'une des revendications 1 à 5, **caractérisée en ce que** l'oligopeptide est un oligopeptide long de 10 à 20 résidus d'acides aminés dont 50 % sont des résidus de lysine et/ou dont environ 50 % sont des résidus de tyrosine, **en ce que** la substance contenant du calcium est de l'hydroxy-apatite, et **en ce que** l'agent de réticulation comprend un éthylèneglycol-oligolactide doté de groupes isocyanate terminaux et du 1,4-diaza-bicyclo[2.2.2]octane (DABCO).

7. Combinaison conforme à l'une des revendications 1 à 6, **caractérisée en ce qu'**elle contient de 10 à 70 % en poids de collagène, de 20 à 90 % en poids de substance contenant du calcium, de 0,05 à 40 % en poids d'agent de réticulation, et de 0 à 40 % en poids d'oligopeptide ou de protéine supplémentaire.

8. Combinaison conforme à l'une des revendications 1 à 7, **caractérisée en ce que** l'un ou plusieurs de ses composants se trouvent séparés les uns des autres, l'un ou plusieurs de ces composants pouvant, en option, être mis dans un solvant.

9. Combinaison conforme à l'une des revendications 1 à 7, **caractérisée en ce que** les composants sont mélangés les uns avec les autres et **en ce que** le collagène est réticulé.

10. Combinaison conforme à la revendication 9, **caractérisée en ce qu'**elle se présente sous la forme d'un os artificiel, d'une pièce d'os, d'un implant ou d'un revêtement d'implant, lequel implant est, en option, une broche, une plaque, une vis, une cheville, une prothèse, un cotyle de hanche artificiel, une "cage" ou un substitut de corps vertébral.

11. Produit médical, **caractérisé en ce qu'**il comprend une combinaison conforme à l'une des revendications 1 à 10.

12. Produit médical conforme à la revendication 11, pour le traitement d'une ostéoporose et/ou la prophylaxie ou le traitement de fractures osseuses, ou pour la fixation d'implants.

13. Utilisation d'une combinaison conforme à la revendication 8 en vue de la fabrication d'un produit médical conçu pour le traitement d'une ostéoporose et/ou la prophylaxie ou le traitement de fractures osseuses, ou pour la fixation d'implants.

14. Utilisation conforme à la revendication 13, **caractérisée en ce que** le produit médical est formulé pour que l'on mélange les composants de la combinaison les uns avec les autres et qu'on les mette en contact avec un os et/ou un implant.

15. Procédé de fabrication *in vitro* d'os artificiel, de pièces d'os, d'implants et/ou de revêtements d'implant, **caractérisé en ce que** l'on met en contact les uns avec les autres les composants d'une combinaison conforme à la revendication 8, et que l'on met le tout sous la forme voulue.
